(19) **Europäisches Patentamt / European Patent Office / Office européen des brevets**

(11) **EP 4 717 305 A1**

(12) **EUROPEAN PATENT APPLICATION**

(43) Date of publication:
**01.04.2026 Bulletin 2026/14**

(21) Application number: **25203539.9**

(22) Date of filing: **22.09.2025**

(51) International Patent Classification (IPC):
***A61N 5/10*** *(2006.01)*

(52) Cooperative Patent Classification (CPC):
**A61N 5/1031**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **25.09.2024 US 202418895899**

(71) Applicant: **Siemens Healthineers AG**
**91301 Forchheim (DE)**

(72) Inventors:
• **KRAUS, Martin**
**90763 Fürth (DE)**

• **ARBERET, Simon**
**Princeton, NJ, 08540 (US)**
• **GAO, Riqiang**
**Plainsboro, NJ, 08536 (US)**
• **GHESU, Florin-Cristian**
**91083 Baiersdorf (DE)**
• **KAMEN, Ali**
**Skillman, NJ, 08558 (US)**

(74) Representative: **HKW Intellectual Property PartGmbB**
**Theresienhöhe 12**
**80339 München (DE)**

(54) **SINGLE-SHOT MANY-FIELD AI DOSE COMPUTATION FOR RADIATION THERAPY**

(57) Systems and methods for determining a multi-field dose for radiation therapy are provided. 1) one or more medical images of a patient and 2) a fluence map for each of a plurality of fields for radiation therapy of the patient are received. Fluence-related information is determined for each of the plurality of fields based on the fluence maps. The fluence-related information for the plurality of fields are aggregated. A multi-field dose for the patient is determined based on the one or more medical images and the aggregated fluence-related information using a machine learning based dose prediction network. The multi-field dose is output.

FIG. 1

100

Receive 1) one or more medical images of a patient and 2) a fluence map for each of a plurality of fields for radiation therapy of the patient
102

Determine fluence-related information for each of the plurality of fields based on the fluence maps
104

Determine an approximated dose for the patient based on the fluence-related information for the plurality of fields
106

Aggregate the fluence-related information for the plurality of fields
108

Determine a multi-field dose for the patient based on the one or more medical images, the aggregated fluence-related information, and the approximated dose using a machine learning based dose prediction network
110

Output the multi-field dose
112

**Description**

TECHNICAL FIELD

The present invention relates generally to AI/ML (artificial intelligence/machine learning) based medical analysis, and in particular to single-shot many-field AI dose computation for radiation therapy.

BACKGROUND

Radiation therapy is a medical treatment that uses high doses of ionizing radiation to kill or damage cancer cells and shrink tumors. Before starting radiation therapy, a detailed treatment plan is created by imaging the patient to locate the tumor and calculating the total radiation dose and how it will be fractionated. Radiation is delivered according to the computed dose by directing radiation fields at the tumor from different angles.

Recently, AI based approaches have been proposed for radiation therapy dose computation. However, such conventional AI based approaches perform dose computation per field. Accordingly, for each field, a coordinate system transformation may need to be performed back and forth, resulting in a linear scaling of runtime with the number of fields. This becomes a runtime problem when the number of fields is high. For example, in VMAT (volumetric modulated arc therapy) plans, the number of fields may be in the order of 180.

BRIEF SUMMARY OF THE INVENTION

In accordance with one or more embodiments, apparatuses, systems, methods, and computer program products for determining a multi-field dose for radiation therapy are provided. 1) one or more medical images of a patient and 2) a fluence map for each of a plurality of fields for radiation therapy of the patient are received. Fluence-related information is determined for each of the plurality of fields based on the fluence maps. The fluence-related information for the plurality of fields are aggregated. A multi-field dose for the patient is determined based on the one or more medical images and the aggregated fluence-related information using a machine learning based dose prediction network. The multi-field dose is output.

In one embodiment, an approximated dose for the patient is determined based on the fluence-related information for the plurality of fields. The multi-field dose for the patient is determined further based on the approximated dose. In one embodiment, an initial multi-field dose is determined based on the one or more medical images and the aggregated fluence-related information using the machine learning based dose prediction network. The initial multi-field dose and the approximated dose are combined to determine the multi-field dose for the patient.

In one embodiment, electron angular fluences are determined using the machine learning based dose prediction network. The multi-field dose for the patient is determined based on the electron angular fluences.

In one embodiment, a direction of the fluence-related information is transformed to coefficient vectors. The coefficient vectors are scaled based on the fluence-related information. The scaled coefficient vectors are combined to provide for the aggregated fluence-related information. The direction of the fluence-related information may be transformed to coefficient vectors for each voxel of the fluence maps. The scaled coefficient vectors may be combined by combining the scaled coefficient vectors into accumulated coefficients for each of the plurality of fields and combining the accumulated coefficients for each of the plurality of fields into combined coefficients.

In one embodiment, the multi-field dose for the patient may be determined further based on a material volume of tissue to be irradiated.

In one embodiment, the fluence-related information comprises uncollided fluence.

These and other advantages of the invention will be apparent to those of ordinary skill in the art by reference to the following detailed description and the accompanying drawings.

BRIEF DESCRIPTION OF THE DRAWINGS

Figure 1 shows a method for determining a dose for radiation therapy of a patient, in accordance with one or more embodiments;

Figure 2 shows a workflow for determining a dose for radiation therapy of a patient, in accordance with one or more embodiments;

Figure 3 shows an exemplary artificial neural network that may be used to implement one or more embodiments;

Figure 4 shows a convolutional neural network that may be used to implement one or more embodiments;

Figure 5 shows a data flow diagram according for using a generative adversarial network to implement one or more embodiments;

Figure 6 shows a schematic structure of a recurrent machine learning model that may be used to implement one or more embodiments; and

Figure 7 shows a high-level block diagram of a computer that may be used to implement one or more embodiments.

DETAILED DESCRIPTION

The present invention generally relates to methods and systems for single-shot many-field AI dose computation for radiation therapy. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system. Further, reference herein to pixels of an image may refer equally to voxels of an image and vice versa.

Embodiments described herein provide for AI/ML based dose computation for radiation therapy. During a pre-processing step, the varying number of field contributions is aggregated into a fixed-dimensional vector per voxel. Conceptually, instead of taking into account the fluence of each field of the radiation beam independently, superposition and linearity can be used to sum fluence contributions from fields of a similar direction at a similar location (assuming either the same spectrum or summing individual spectral components). A split dose computation is proposed in two phases. In a first phase, contributions from fluences from different fields are aggregated/superimposed. This aggregation is performed per-field. In general, the field contributions (e.g., strength and direction) are aggregated to a fixed-dimensional space where they can be superimposed. In a second phase, the aggregated information is input into a machine learning based model for predicting the resulting dose.

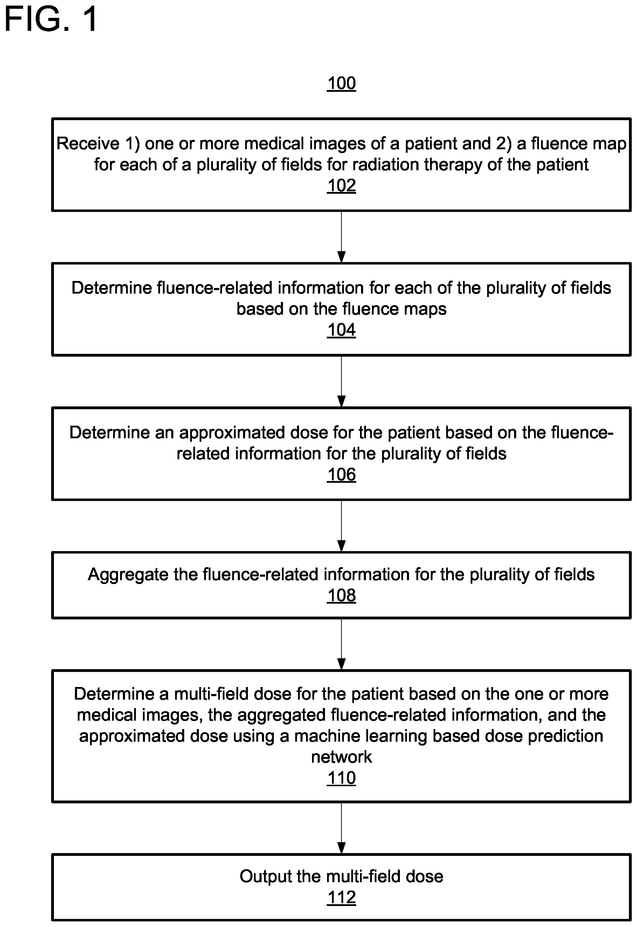

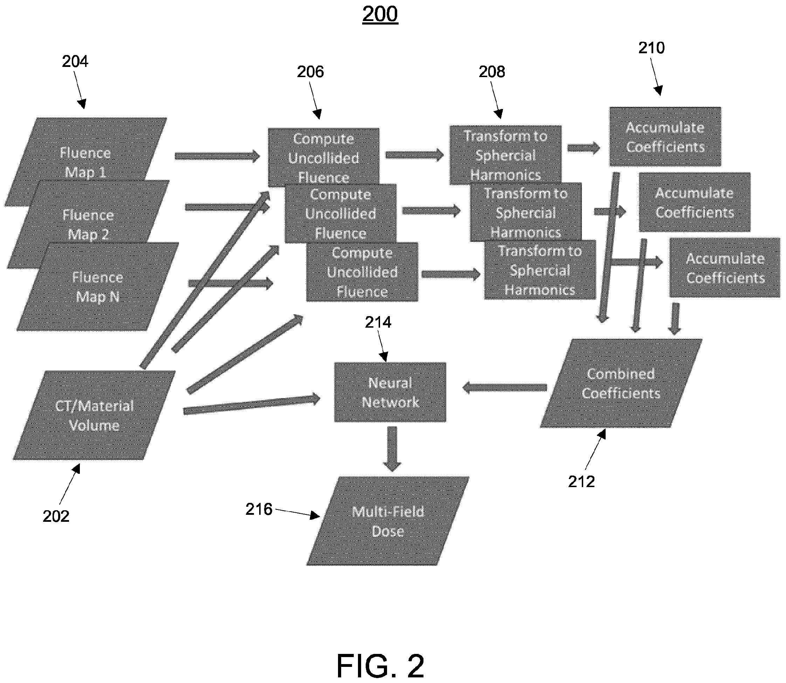

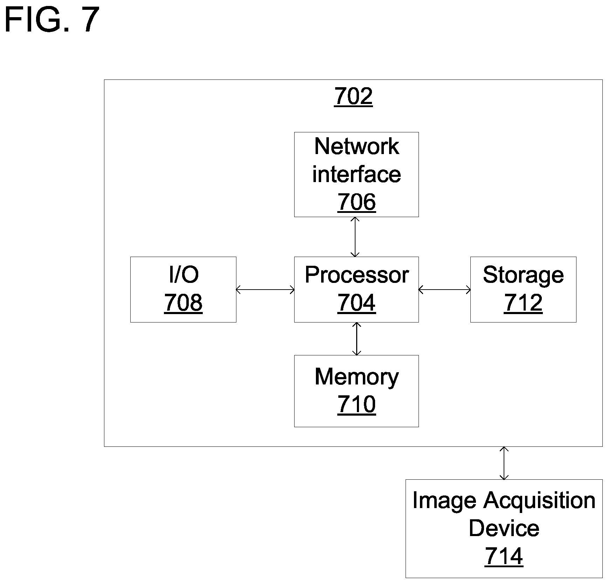

Figure 1 shows a method 100 for determining a dose for radiation therapy of a patient, in accordance with one or more embodiments. The steps and sub-steps of method 100 may be performed by one or more suitable computing devices, such as, e.g., computer 702 of Figure 7. Figure 2 shows a workflow 200 for determining a dose for radiation therapy of a patient, in accordance with one or more embodiments. Figure 1 and Figure 2 will be described together.

At step 102 of Figure 1, 1) one or more medical images of a patient and 2) a fluence map for each of a plurality of fields for radiation therapy of the patient are received. In one example, as shown in workflow 200 of Figure 2, the one or more medical images is CT (computed tomography) image 202 and the fluence maps are fluence maps 204.

The one or more medical images depict an anatomical object to be irradiated during radiation therapy. For example, the anatomical object may comprise tumors or other abnormalities, bones, vessels, organs, or any other anatomical object of interest. In one embodiment, the one or more medical images comprise CT images. However, the one or more medical images may comprise any other suitable modality, such as, e.g., MRI (magnetic resonance imaging), US (ultrasound), x-ray, or any other medical imaging modality or combinations of medical imaging modalities. The one or more medical images may comprise 2D (two dimensional) images and/or 3D (three dimensional) volumes, and may comprise a single medical image or a plurality of medical images. In one embodiment, instead or in addition to the one or more medical images, a material volume of tissue to be irradiated during the radiation therapy may be received.

The fluence maps represent the intensity of radiation across a particular field. The field refers to the specific areas where radiation beams are directed during radiation therapy and is defined by the geometry, intensity, and direction of the radiation beam. The fluence maps may be created during treatment planning using a treatment planning system, e.g., according to any well-known approach.

The one or more medical images and/or the fluence maps may be received, for example, by directly receiving the one or more medical images from an image acquisition device (e.g., image acquisition device 714 of Figure 7) as the medical images are acquired, by loading the one or more medical images and/or the fluence maps from a storage or memory of a computer system (e.g., storage 712 or memory 710 of computer 702 of Figure 7), or by receiving the one or more medical images and/or the fluence maps from a remote computer system (e.g., computer 702 of Figure 7). Such a computer system or remote computer system may comprise one or more patient databases, such as, e.g. , an EHR (electronic health record), EMR (electronic medical record), PHR (personal health record), HIS (health information system), RIS (radiology information system), PACS (picture archiving and communication system), LIMS (laboratory information management system), or any other suitable database or system.

At step 104 of Figure 1, fluence-related information for each of the plurality of fields is determined based on the fluence maps. In one embodiment, the fluence-related information is uncollided fluence. In one example, as shown in workflow 200 of Figure 2, uncollided fluence 206 is computed for each respective fluence map 204.

Uncollided fluence refers to fluence that is not absorbed by the patient. The uncollided fluence may be determined for each respective field of the plurality of fields for each voxel of the fluence map for the respective field according to any suitable approach. In one embodiment, the uncollided fluence may be determined using a radiation model based on known material constants. An example of a radiation model is the Beer-Lambert model as defined according to Equation (1):

$$I(d, E_1, E_2) \rightarrow \int_{E1}^{E2} I(0, E) \cdot e^{-\int_0^d \mu(E,s)ds} dE \qquad (1)$$

where $E_1$ and $E_2$ is a certain energy range, $I(0, E)$ is the source intensity at energy $E$, $d$ is depth along the ray, $I(d, E_1, E_2)$ is the uncollided fluence at depth d for energy interval $[E_1, E_2]$, and $\mu(E, s)$ represent material absorption coefficients at a location s and at energy E.

Given that a typical dose operates on N distinct materials, Equation (1) may be modified as follows in Equation (2):

$$I(d, E_1, E_2) \rightarrow \int_{E1}^{E2} I(0, E) \cdot e^{-\int_{n=0}^N -d_n \mu_n(E)} dE \qquad (2)$$

where $d_n$ corresponds to the length of the path up to depth $d$ that is through material with index $n$ and $\mu_n(E)$ is the absorption and scattering (i.e., what collides) coefficient for material $n$ at energy $E$. For a certain fixed spectrum, coefficient $\mu_n(E)$ can be reduced to $\mu_n$. The coefficients can either be known from existing material databases or can be estimated for a certain scenario of material and spectral decomposition. A more sophisticated version of Equation (2) may be used where the path length through each voxel of the fluence maps is multiplied by the density of the voxel times the coefficient $\mu_n(E)$ from the material of the voxel, and integrated over the whole path.

The fluence-related information may comprise any other suitable information relating to the fluence. For example, in one embodiment, the fluence-related information may comprise the fluence for each of the plurality of fields. In other embodiments, the fluence-related information may comprise additional information of the radiation spectrum, the relative position (e.g., the (x, y) coordinate) of the ray on the fluence map, and or information relating to the distance of the ray from the center of the beam on the fluence map.

At step 106 of Figure 1, optionally, an approximated dose for the patient is determined based on the fluence-related information for the plurality of fields. In one embodiment, where the fluence-related information comprises uncollided fluences, the uncollided fluences are directly converted to the approximated dose based on the combined absorption and scattering of that material. The approximated dose may be determined according to the radiation model of Equation (3):

$$D(d, E_1, E_2) = I(d, E_1, E_2) \cdot \left(1 - e^{-\mu_n(E_1, E_2)}\right) \cdot s \qquad (3)$$

where $D(d, E_1, E_2)$ is the approximated dose at depth $d$ for energy interval $[E_1, E_2]$, s is a scaling factors, and $\mu_n(E_1, E_2)$ is an absorption factor for material $n$ that is appropriate for energy interval $[E_1, E_2]$. $\mu_n$ and s can be estimated, for example, using L1 loss with respect to a target final dose. Equation (3) assumes only first order interaction and no scattering. This model will correspond best to the final dose if the coefficients are more accurate. These parameters may be fixed while the machine learning based prediction network (utilized at step 110 of Figure 1) is trained. The approximated dose may be determined using any other suitable approach. For example, a fast optimizer type dose method may be used for determining the approximated dose. In another example, Equation (3) may be combined with a convolution kernel for determining the approximated dose.

At step 108 of Figure 1, the fluence-related information for the plurality of fields are aggregated. The fluence-related information may be aggregated according to any suitable approach. In one embodiment, the uncollided fluences are aggregated per-voxel using spherical harmonics by projecting the fluence-related information onto a 3D computational grid and transforming the projected fluence-related information into spherical harmonic coefficients. In one example, as shown in workflow 200 of Figure 2, uncollided fluences 206 are respectively transformed to spherical harmonics 208 and the coefficients 210 of the spherical harmonics are respectively accumulated for each fluence map 204 (i.e., for each field). The accumulated coefficients 210 for each fluence map 204 are combined to provide for combined coefficients 212.

To aggregate the uncollided fluences using spherical harmonics, for a given field and for a given voxel corresponding to the normalized direction from the source, uncollided fluence $I(d, E_1, E_2)$ is mapped into the spherical harmonics domain by transforming the direction of uncollided fluence $I(d, E_1, E_2)$ to coefficient vectors and scaling the coefficient vectors with uncollided fluence $I(d, E_1, E_2)$. The scaled coefficient vectors thus account for a certain uncollided fluence from a certain direction impinging at (x,y,z) (i.e., at a certain spectrum or energy bin). Given that coefficient vectors are of fixed-dimension, aggregation of the uncollided fluence $I(d, E_1, E_2)$ can be performed by combining (e.g., adding) the coefficient vectors. Specifically, for each respective fluence map, the coefficient vectors for each voxel of the respective fluence map are combined into an accumulated coefficient vector for the respective fluence map and the accumulated coefficient vectors for the fluence maps are combined into a combined coefficient vector. While described in terms of aggregating the uncollided fluences using spherical harmonics, it should be understood that the same or similar approach may be used for aggregating any of the fluence-related information using spherical harmonics.

At step 110 of Figure 1, a multi-field dose for the patient is determined based on the one or more medical images, the aggregated fluence-related information, and (optionally) the approximated dose using a machine learning based dose prediction network. In one embodiment, as shown in workflow 200 of Figure 2, multi-field dose 216 is determined based on CT image 202 and combined coefficients 212 using neural network 214.

The machine learning based dose prediction network receives as input the one or more medical images, the aggregated fluence-related information (e.g., the coefficients), and (optionally) the approximated dose and generates as output the multi-field dose. Using the aggregated fluence-related information as input simplifies the network prediction task. It takes care of the first order long range interaction between the source and the current voxel. Additionally, further order interactions tend to be shorter range and reduced in influence on the final dose. Therefore, the machine learning based dose prediction network can have a smaller receptive field and less capacity for the same accuracy when using the aggregated uncollided fluence as input. In one embodiment, the material volume of tissue to be irradiated for the fluence maps may be received as input to the machine learning based dose prediction network instead of or in addition to the one or more medical images.

The machine learning based dose prediction network may be implemented according to any suitable machine learning based architecture. For example, where the voxels are sampled in a regular grid, a normal image-to-image network may be used, such as, e.g., a U-Net. The machine learning based dose prediction network is trained during a prior offline or training stage using a training dataset comprising medical images, aggregated fluence-related information, and (optionally) approximated doses paired with multi-field doses. Once trained, the machine learning based dose prediction network is applied during an online or inference stage, e.g., to perform step 110 of Figure 1.

In one embodiment, the machine learning based dose prediction network generates as output an initial multi-field dose and the initial multi-field dose is combined with the approximated dose to determine the final multi-field dose determined as step 110.

In one embodiment, instead of the machine learning based dose prediction network generating the multi-field dose, the machine learning based dose prediction network instead determines the electron angular fluences per voxel and the final multi-field dose may be calculated according to Equation (4):

$$D_i = \int\limits_0^\infty dE \int\limits_{4\pi} d\widehat{\Omega} \frac{\sigma_{ED}^e(\vec{r}, E)}{\rho} \Phi^e(\vec{r}, E, \widehat{\Omega}) \tag{4}$$

Electron angular fluence refers to the distribution of electron particles as they pass through a given field at specific angles. Instead of the machine learning based dose prediction network $NN(x)$ predicting multi-field dose $D_i$, the structure of Equation (4) can be incorporated in different ways. For example, the division of $\rho$ can be removed, such that $D = NN(x)/\rho$. Furthermore, $\Phi^e(\vec{r}, E, \widehat{\Omega})$ can be predicted by the network in terms of spherical harmonics representation. The rest of Equation (4) can then be computed in post-processing. Cross-section information $\sigma_{ED}^e(\vec{r}, E)$ is available in principle, but can also be regressed. The integration over energy can be performed according to any suitable approach.

At step 112 of Figure 1, the multi-field dose is output. For example, the multi-field dose can be output by displaying the multi-field dose on a display device of a computer system (e.g., I/O 708 of computer 702 of Figure 7), storing the multi-field dose on a memory or storage of a computer system (e.g., memory 710 or storage 712 of computer 702 of Figure 7), or by transmitting the multi-field dose to a remote computer system (e.g., computer 702 of Figure 7).

In one embodiment, a specific data augmentation strategy for augmenting the training dataset for training the machine learning based dose prediction network is provided by dropping out a random number of fields from a multi-field dose plan. For a 180-field VMAT (volumetric modulated arc therapy) plan, this generates a very large number of permutations of input/target pairs. Individual field fluences can also randomly scaled by a factor around 1. Given that target dose is stored per field, due to linearity, a combined target dose can be computed as a linear combination of the field doses. In the machine learning based dose prediction network is trained in such a way, it would learn the actual dose contributions per field to succeed. This means that it is more likely that the network also works with new types of plans, such as, e.g., hybrid approaches between IMRT (intensity-modulated radiation therapy) and VMAT, where there are some IMRT fields and one or more smaller arcs of VMAT like fields.

In one embodiment, the spectrum of the fluences can be account for in different ways. First, it can be assumed that spectrum is always the same. Second, the spectrum can be subdivided into sub-spectra. This can be performed as simple binning or as a more sophisticated linear decomposition such as, e.g., using PCA (principal component analysis). Given a certain number of bins or spectral coefficients, there is one set of spherical harmonics (or other) coefficients per bin. These multiple coefficients can be fed into a single network as channels. Alternatively, given linearity of dose in fluence and over spectrum, multiple machine learning based dose prediction networks can be used, where each handles a single spectra bin or coefficients or sets therefore. Network outputs can then be linearly combined to the final full spectrum dose.

Advantageously, embodiments described herein provide better speed and accuracy tradeoff through the single shot nature that allows the use of linear superposition in the input stage to reduce the number of computations needed. Further, through incorporation of physical knowledge, through using uncollided fluences (and other fluence-related information) analogues as input, and optionally using electron angular fluence as output instead of dose, this frees the machine learning based dose prediction network from having to perform long spatial context tasks (uncollided fluence) for the input stage. For the output stage, physics-based post processing of the output can be added to gain functionality, such as, e.g., being able to compute dose to water as well as dose to tissue.

Embodiments described herein are described with respect to the claimed systems as well as with respect to the claimed methods. Features, advantages or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Furthermore, certain embodiments described herein are described with respect to methods and systems utilizing trained machine learning models, as well as with respect to methods and systems for providing trained machine learning models. Features, advantages or alternative embodiments herein can be assigned to the other claimed objects and vice versa. In other words, claims and embodiments for providing trained machine learning models can be improved with features described or claimed in the context of utilizing trained machine learning models, and vice versa. In particular, datasets used in the methods and systems for utilizing trained machine learning models can have the same properties and features as the corresponding datasets used in the methods and systems for providing trained machine learning models, and the trained machine learning models provided by the respective methods and systems can be used in the methods and systems for utilizing the trained machine learning models.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function."

In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used.

In particular, a machine learning model, such as, e.g., the machine learning based dose prediction network utilized at step 110 of Figure 1 and neural network 214 of Figure 2, can comprise, for example, a neural network, a support vector machine, a decision tree and/or a Bayesian network, and/or the machine learning model can be based on, for example, k-means clustering, Q-learning, genetic algorithms and/or association rules. In particular, a neural network can be, e.g., a deep neural network, a convolutional neural network or a convolutional deep neural network. Furthermore, a neural network can be, e.g., an adversarial network, a deep adversarial network and/or a generative adversarial network.

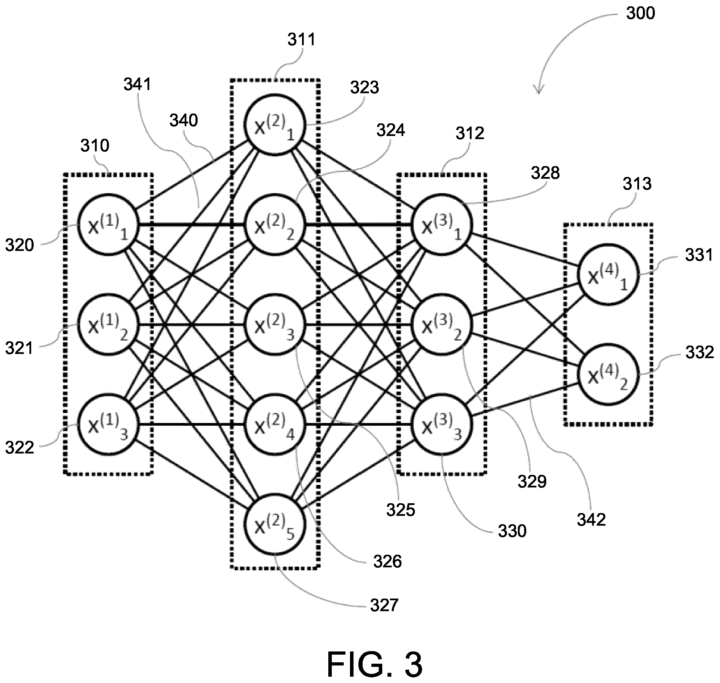

Figure 3 shows an embodiment of an artificial neural network 300 that may be used to implement one or more machine learning models described herein. Alternative terms for "artificial neural network" are "neural network", "artificial neural net" or "neural net".

The artificial neural network 300 comprises nodes 320, ..., 332 and edges 340, ..., 342, wherein each edge 340, ..., 342 is a directed connection from a first node 320, ..., 332 to a second node 320, ..., 332. In general, the first node 320, ..., 332 and the second node 320, ..., 332 are different nodes 320, ..., 332, it is also possible that the first node 320, ..., 332 and the second node 320, ..., 332 are identical. For example, in Figure 3 the edge 340 is a directed connection from the node 320 to the node 323, and the edge 342 is a directed connection from the node 330 to the node 332. An edge 340, ..., 342 from a first node 320, ..., 332 to a second node 320, ..., 332 is also denoted as "ingoing edge" for the second node 320, ..., 332 and as "outgoing edge" for the first node 320, ..., 332.

In this embodiment, the nodes 320, ..., 332 of the artificial neural network 300 can be arranged in layers 310, ...,

313, wherein the layers can comprise an intrinsic order introduced by the edges 340, ..., 342 between the nodes 320, ..., 332. In particular, edges 340, ..., 342 can exist only between neighboring layers of nodes. In the displayed embodiment, there is an input layer 310 comprising only nodes 320, ..., 322 without an incoming edge, an output layer 313 comprising only nodes 331, 332 without outgoing edges, and hidden layers 311, 312 in-between the input layer 310 and the output layer 313. In general, the number of hidden layers 311, 312 can be chosen arbitrarily. The number of nodes 320, ..., 322 within the input layer 310 usually relates to the number of input values of the neural network, and the number of nodes 331, 332 within the output layer 313 usually relates to the number of output values of the neural network.

In particular, a (real) number can be assigned as a value to every node 320, ..., 332 of the neural network 300. Here, $x^{(n)}_i$ denotes the value of the i-th node 320, ..., 332 of the n-th layer 310, ..., 313. The values of the nodes 320, ..., 322 of the input layer 310 are equivalent to the input values of the neural network 300, the values of the nodes 331, 332 of the output layer 313 are equivalent to the output value of the neural network 300. Furthermore, each edge 340, ..., 342 can comprise a weight being a real number, in particular, the weight is a real number within the interval [-1, 1] or within the interval [0, 1]. Here, $w^{(m,n)}_{i,j}$ denotes the weight of the edge between the i-th node 320, ..., 332 of the m-th layer 310, ..., 313 and the j-th node 320, ..., 332 of the n-th layer 310, ..., 313. Furthermore, the abbreviation $w^{(n)}_{i,j}$ is defined for the weight $w^{(n,n+1)}_{i,j}$.

In particular, to calculate the output values of the neural network 300, the input values are propagated through the neural network. In particular, the values of the nodes 320, ..., 332 of the (n+1)-th layer 310, ..., 313 can be calculated based on the values of the nodes 320, ..., 332 of the n-th layer 310, ..., 313 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g., the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 310 are given by the input of the neural network 300, wherein values of the first hid-den layer 311 can be calculated based on the values of the input layer 310 of the neural network, wherein values of the second hidden layer 312 can be calculated based in the values of the first hidden layer 311, etc.

In order to set the values $w^{(m,n)}_{i,j}$ for the edges, the neural network 300 has to be trained using training data. In particular, training data comprises training input data and training output data (denoted as $t_i$). For a training step, the neural network 300 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 300 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein γ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta^{(n)}_j = \left(x^{(n+1)}_j - t^{(n+1)}_j\right) \cdot f'\left(x^{(n)}_i \cdot w^{(n)}_{i,j}\right)$$

if the (n+1)-th layer is the output layer 313, wherein f is the first derivative of the activation function, and $t^{(n+1)}_j$ is the comparison training value for the j-th node of the output layer 313.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / image, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

By using convolutional neural networks input images can be processed in a very efficient way, because a convolution operation based on different kernels can extract various image features, so that by adapting the weights of the convolution kernel the relevant image features can be found during training. Furthermore, based on the weight-sharing in the convolutional kernels less parameters need to be trained, which prevents overfitting in the training phase and allows to have faster training or more layers in the network, improving the performance of the network.

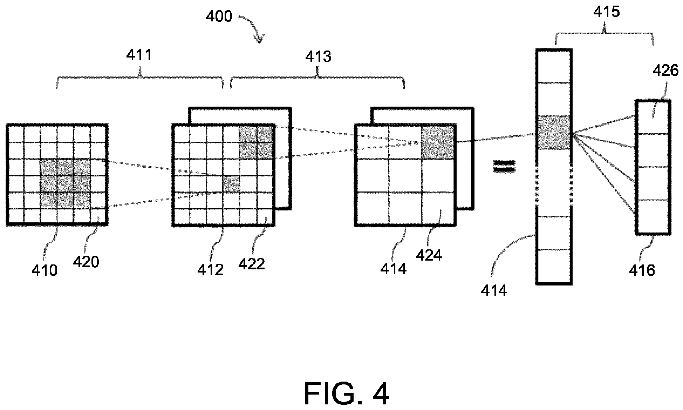

Figure 4 shows an embodiment of a convolutional neural network 400 that may be used to implement one or more machine learning models described herein. In the displayed embodiment, the convolutional neural network comprises 400 an input node layer 410, a convolutional layer 411, a pooling layer 413, a fully connected layer 414 and an output node layer 416, as well as hidden node layers 412, 414. Alternatively, the convolutional neural network 400 can comprise several convolutional layers 411, several pooling layers 413 and several fully connected layers 415, as well as other types of layers. The order of the layers can be chosen arbitrarily, usually fully connected layers 415 are used as the last layers before the output layer 416.

In particular, within a convolutional neural network 400 nodes 420, 422, 424 of a node layer 410, 412, 414 can be considered to be arranged as a d-dimensional matrix or as a d-dimensional image. In particular, in the two-dimensional case the value of the node 420, 422, 424 indexed with i and j in the n-th node layer 410, 412, 414 can be denoted as x(n)[i, j]. However, the arrangement of the nodes 420, 422, 424 of one node layer 410, 412, 414 does not have an effect on the calculations executed within the convolutional neural network 400 as such, since these are given solely by the structure and the weights of the edges.

A convolutional layer 411 is a connection layer between an anterior node layer 410 (with node values x(n-1)) and a posterior node layer 412 (with node values x(n)). In particular, a convolutional layer 411 is characterized by the structure and the weights of the incoming edges forming a convolution operation based on a certain number of kernels. In particular, the structure and the weights of the edges of the convolutional layer 411 are chosen such that the values x(n) of the nodes 422 of the posterior node layer 412 are calculated as a convolution x(n) = K * x(n-1) based on the values x(n-1) of the nodes 420 anterior node layer 410, where the convolution * is defined in the two-dimensional case as

$$x_k^{(n)}[i,j] = \left(K *x^{(n\cdot 1)}\right)[i,j] = \sum_{i'}\sum_{j'} K\ [i',j'] \cdot x^{(n\cdot 1)}[i\text{-}i',\ j\text{-}j'].$$

Here the kernel K is a d-dimensional matrix (in this embodiment, a two-dimensional matrix), which is usually small compared to the number of nodes 420, 422 (e.g., a 3x3 matrix, or a 5x5 matrix). In particular, this implies that the weights of the edges in the convolution layer 411 are not independent, but chosen such that they produce said convolution equation. In particular, for a kernel being a 3x3 matrix, there are only 9 independent weights (each entry of the kernel matrix corresponding to one independent weight), irrespectively of the number of nodes 420, 422 in the anterior node layer 410 and the posterior node layer 412.

In general, convolutional neural networks 400 use node layers 410, 412, 414 with a plurality of channels, in particular, due to the use of a plurality of kernels in convolutional layers 411. In those cases, the node layers can be considered as (d+1)-dimensional matrices (the first dimension indexing the channels). The action of a convolutional layer 411 is then a two-dimensional example defined as

$$x^{(n)_b}\ [i,j] = \sum_{a} K_{a,b} *x^{(n\cdot 1)_a}[i,j] = \sum_{a}\sum_{i'}\sum_{j'} K_{a,b}[i',j'] \cdot x^{(n\cdot 1)_a}\left[i\text{-}i',j\text{-}j'\right]$$

where $x^{(n-1)_a}$ corresponds to the a-th channel of the anterior node layer 410, $x^{(n)_b}$ corresponds to the b-th channel of the posterior node layer 412 and $K_{a,b}$ corresponds to one of the kernels. If a convolutional layer 411 acts on an anterior node layer 410 with A channels and outputs a posterior node layer 412 with B channels, there are A·B independent d-dimensional kernels $K_{a,b}$.

In general, in convolutional neural networks 400 activation functions are used. In this embodiment re ReLU (acronym for "Rectified Linear Units") is used, with R(z) = max(0, z), so that the action of the convolutional layer 411 in the two-dimensional example is

$$x^{(n)_b}\ [i,j] = R\left(\sum_{a}\left(K_{a,b} * x^{(n-1)a}\right)[i,j]\right) = R\left(\sum_{a}\sum_{i'}\sum_{j'} K_{a,b}[i',j'] \cdot x^{(n-1)a}[i-i',j-j']\right)$$

It is also possible to use other activation functions, e.g., ELU (acronym for "Exponential Linear Unit"), LeakyReLU, Sigmoid, Tanh or Softmax.

In the displayed embodiment, the input layer 410 comprises 36 nodes 420, arranged as a two-dimensional 6x6 matrix. The first hidden node layer 412 comprises 72 nodes 422, arranged as two two-dimensional 6x6 matrices, each of the two matrices being the result of a convolution of the values of the input layer with a 3x3 kernel within the convolutional layer 411. Equivalently, the nodes 422 of the first hidden node layer 412 can be interpreted as arranged as a three-dimensional 2x6x6 matrix, wherein the first dimension correspond to the channel dimension.

The advantage of using convolutional layers 411 is that spatially local correlation of the input data can exploited by enforcing a local connectivity pattern between nodes of adjacent layers, in particular by each node being connected to only a small region of the nodes of the preceding layer.

A pooling layer 413 is a connection layer between an anterior node layer 412 (with node values x(n-1)) and a posterior node layer 414 (with node values x(n)). In particular, a pooling layer 413 can be characterized by the structure and the weights of the edges and the activation function forming a pooling operation based on a nonlinear pooling function f. For example, in the two-dimensional case the values x(n) of the nodes 424 of the posterior node layer 414 can be calculated based on the values x(n-1) of the nodes 422 of the anterior node layer 412 as

$$\mathrm{x}^{(n)b}[\mathrm{i},\mathrm{j}] = \mathrm{f}(\mathrm{x}^{(n\text{-}1)}[\mathrm{id}_1, \mathrm{jd}_2], \ldots, \mathrm{x}^{(n-1)b}[(i+1)\mathrm{d}_1 \cdot 1, \, (j+1)\mathrm{d}_2 \cdot 1])$$

In other words, by using a pooling layer 413 the number of nodes 422, 424 can be reduced, by re-placing a number d1·d2 of neighboring nodes 422 in the anterior node layer 412 with a single node 422 in the posterior node layer 414 being calculated as a function of the values of said number of neighboring nodes. In particular, the pooling function f can be the max-function, the average or the L2-Norm. In particular, for a pooling layer 413 the weights of the incoming edges are fixed and are not modified by training.

The advantage of using a pooling layer 413 is that the number of nodes 422, 424 and the number of parameters is reduced. This leads to the amount of computation in the network being reduced and to a control of overfitting.

In the displayed embodiment, the pooling layer 413 is a max-pooling layer, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

In general, the last layers of a convolutional neural network 400 are fully connected layers 415. A fully connected layer 415 is a connection layer between an anterior node layer 414 and a posterior node layer 416. A fully connected layer 413 can be characterized by the fact that a majority, in particular, all edges between nodes 414 of the anterior node layer 414 and the nodes 416 of the posterior node layer are present, and wherein the weight of each of these edges can be adjusted individually.

In this embodiment, the nodes 424 of the anterior node layer 414 of the fully connected layer 415 are displayed both as two-dimensional matrices, and additionally as non-related nodes (indicated as a line of nodes, wherein the number of nodes was reduced for a better presentability). This operation is also denoted as "flattening". In this embodiment, the number of nodes 426 in the posterior node layer 416 of the fully connected layer 415 smaller than the number of nodes 424 in the anterior node layer 414. Alternatively, the number of nodes 426 can be equal or larger.

Furthermore, in this embodiment the Softmax activation function is used within the fully connected layer 415. By applying the Softmax function, the sum the values of all nodes 426 of the output layer 416 is 1, and all values of all nodes 426 of the output layer 416 are real numbers between 0 and 1. In particular, if using the convolutional neural network 400 for categorizing input data, the values of the output layer 416 can be interpreted as the probability of the input data falling into one of the different categories.

In particular, convolutional neural networks 400 can be trained based on the backpropagation algorithm. For preventing overfitting, methods of regularization can be used, e.g., dropout of nodes 420, ..., 424, stochastic pooling, use of artificial data, weight decay based on the L1 or the L2 norm, or max norm constraints.

According to an aspect, the machine learning model may comprise one or more residual networks (ResNet). In particular, a ResNet is an artificial neural network comprising at least one jump or skip connection used to jump over at least one layer of the artificial neural network. In particular, a ResNet may be a convolutional neural network comprising one or more skip connections respectively skipping one or more convolutional layers. According to some examples, the ResNets may be represented as m-layer ResNets, where m is the number of layers in the corresponding architecture and, according to some examples, may take values of 34, 50, 101, or 152. According to some examples, such an m-layer ResNet may respectively comprise (m-2)/2 skip connections.

A skip connection may be seen as a bypass which directly feeds the output of one preceding layer over one or more bypassed layers to a layer succeeding the one or more bypassed layers. Instead of having to directly fit a desired mapping, the bypassed layers would then have to fit a residual mapping "balancing" the directly fed output.

Fitting the residual mapping is computationally easier to optimize than the directed mapping. What is more, this alleviates the problem of vanishing/exploding gradients during optimization upon training the machine learning models: if a bypassed layer runs into such problems, its contribution may be skipped by regularization of the directly fed output. Using ResNets thus brings about the advantage that much deeper networks may be trained.

A generative adversarial model (an acronym is GA model) comprises a generative function and a discriminative function, wherein the generative function creates synthetic data, and the discriminative function distinguishes between synthetic and real data. By training the generative function and/or the discriminative function on the one hand the generative function is configured to create synthetic data which is incorrectly classified by the discriminative function as real, on the other hand the discriminative function is configured to distinguish between real data and synthetic data generated by the generative function. In the notion of game theory, a generative adversarial model can be interpreted as a zero-sum game. The training of the generative function and/or of the discriminative function is based, in particular, on the minimization of a cost function.

By using a GA model, based on a set of training data synthetic data can be generated that has the same characteristics as the training data set. The training of the GA model can be based on data not being annotated (unsupervised learning), so that there is low effort in training a GA model.

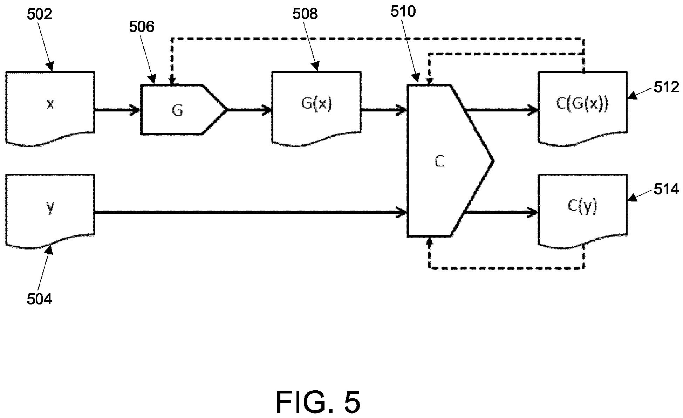

Figure 5 shows a data flow diagram according to an embodiment for using a generative adversarial network for creating synthetic output data G(x) 508 based on input data x 502 that is indistinguishable from real output data y 504, in accordance with one or more embodiments. The synthetic output data G(x) 508 has the same structure as the real output data y 504, but its content is not derived from real world data.

The generative adversarial network comprises a generator function G 506 and a classifier function C 510 which are trained jointly. The task of the generator function G 506 is to provide realistic synthetic output data G(x) 508 based on input data x 502, and the task of the classifier function C 510 is to distinguish between real output data y 504 and synthetic output data G(x) 508. In particular, the output of the classifier function C 510 is a real number between 0 and 1 corresponding to the probability of the input value being real data, so that an ideal classifier function would calculate an output value of $C(y)$ 514 $\approx$ 1 for real data y 504 and $C(G(x))$ 512 $\approx$ 0 for synthetic data G(x) 508.

Within the training process, parameters of the generator function G 506 are adapted so that the synthetic output data G(x) 508 has the same characteristics as real output data y 504, so that the classifier function C 510 cannot distinguish between real and synthetic data anymore. At the same time, parameters of the classifier function C 510 are adapted so that it distinguishes between real and synthetic data in the best possible way. Here, the training relies on pairs comprising input data x 502 and the corresponding real output data y 504. Within a single training step, the generator function G 506 is applied to the input data x 502 for generating synthetic output data G(x) 508. Furthermore, the classifier function C 510 is applied to the real output data y 504 for generating a first classification result C(y) 514. Additionally, the classifier function C 510 is applied to the synthetic output data G(x) 508 for generating a second classification result C(G(x)) 512.

Adapting the parameters of the generative function G 506 and the classifier function C 510 is based on minimizing a cost function by using the backpropagation algorithm, respectively. In this embodiment, the cost function $K_C$ for the classifier function C 510 is $K_C \propto - BCE(C(y), 1) - BCE(C(G(x)), 0)$, wherein BCE denotes the binary cross entropy defined as $BCE(z, z') = z' \cdot \log(z) + (1 - z') \cdot \log(1 - z)$. By using this cost function, both wrongly classifying real output data as synthetic (indicated by $C(y) \approx 0$) and wrongly classifying synthetic output data as real (indicated as $C(G(x))$ 512 $\approx$ 1) increases the cost function $K_C$ to be minimized. Furthermore, the cost function $K_G$ for the generator function G 506 is $K_G \propto - BCE(C(G(x)), 1) = - \log(C(G(x))$. By using this cost function, correctly classified synthetic output data (indicated as $C(G(x))$ 512 $\approx$ 0) leads to an increase of the cost function $K_G$ to be minimized.

In particular, a recurrent machine learning model is a machine learning model whose output does not only depend on the input value and the parameters of the machine learning model adapted by the training process, but also on a hidden state vector, wherein the hidden state vector is based on previous inputs used on for the recurrent machine learning model. In particular, the recurrent machine learning model can comprise additional storage states or additional structures that incorporate time delays or comprise feedback loops.

In particular, the underlying structure of a recurrent machine learning model can be a neural network, which can be denoted as recurrent neural network. Such a recurrent neural network can be described as an artificial neural network where connections between nodes form a directed graph along a temporal sequence. In particular, a recurrent neural network can be interpreted as directed acyclic graph. In particular, the recurrent neural network can be a finite impulse recurrent neural network or an infinite impulse recurrent neural network (wherein a finite impulse network can be unrolled and replaced with a strictly feedforward neural network, and an infinite impulse network cannot be unrolled and replaced with a strictly feedforward neural network).

In particular, training a recurrent neural network can be based on the BPTT algorithm (acronym for "backpropagation through time"), on the RTRL algorithm (acronym for "real-time recurrent learning") and/or on genetic algorithms.

By using a recurrent machine learning model input data comprising sequences of variable length can be used. In particular, this implies that the method cannot be used only for a fixed number of input datasets (and needs to be trained differently for every other number of input datasets used as input), but can be used for an arbitrary number of input datasets. This implies that the whole set of training data, independent of the number of input datasets contained in different sequences, can be used within the training, and that training data is not reduced to training data corresponding to a certain number of successive input datasets.

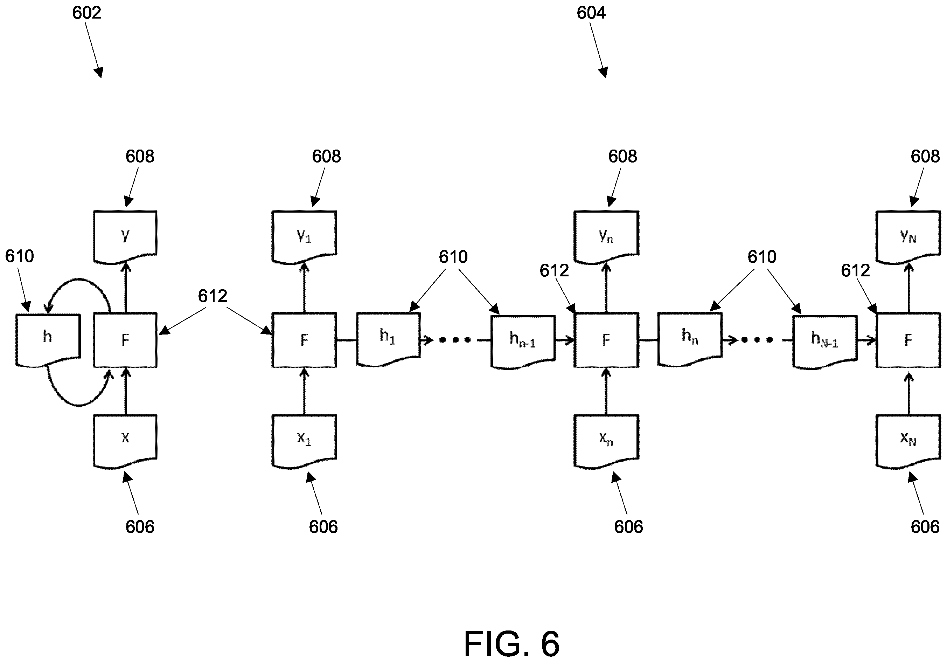

Figure 6 shows the schematic structure of a recurrent machine learning model F, both in a recurrent representation 602 and in an unfolded representation 604, that may be used to implement one or more machine learning models described herein. The recurrent machine learning model takes as input several input datasets x, $x_1$, ..., $x_N$ 606 and creates a corresponding set of output datasets y, $y_1$, ..., $y_N$ 608. Furthermore, the output depends on a so-called hidden vector h, $h_1$, ..., $h_N$ 610, which implicitly comprises information about input datasets previously used as input for the recurrent machine learning model F 612. By using these hidden vectors h, $h_1$, ..., $h_N$ 610, a sequentiality of the input datasets can be leveraged.

In a single step of the processing, the recurrent machine learning model F 612 takes as input the hidden vector $h_{n-1}$ created within the previous step and an input dataset $x_n$. Within this step, the recurrent machine learning model F generates as output an updated hidden vector $h_n$ and an output dataset $y_n$. In other words, one step of processing calculates $(y_n, h_n) = F(x_n, h_{n-1})$, or by splitting the recurrent machine learning model F 612 into a part F(y) calculating the output data and F(h) calculating the hidden vector, one step of processing calculates $y_n = F^{(y)}(x_n, h_{n-1})$ and $h_n = F^{(h)}(x_n, h_{n-1})$. For the first processing step, $h_0$ can be chosen randomly or filled with all entries being zero. The parameters of the recurrent machine learning model F 612 that were trained based on training datasets before do not change between the different processing steps.

In particular, the output data and the hidden vector of a processing step depend on all the previous input datasets used in the previous steps. $y_n = F^{(y)}(x_n, F^{(h)}(x_{n-1}, h_{n-2}))$ and $h_n = F(h)(x_n, F^{(h)}(x_{n-1}, h_{n-2}))$.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatuses, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatuses, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatuses, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 702 that may be used to implement systems, apparatuses, and methods described herein is depicted in Figure 7. Computer 702 includes a processor 704 operatively coupled to a data storage device 712 and a memory 710. Processor 704 controls the overall operation of computer 702 by executing computer program instructions that define such operations. The computer program instructions may be stored in data storage device 712, or other computer readable medium, and loaded into memory 710 when execution of the computer program instructions is desired. Thus, the method and workflow steps or functions of Figures 1 or 2 can be defined by the computer program instructions stored in memory 710 and/or data storage device 712 and controlled by processor 704 executing the computer program instructions. For example, the computer program instructions can be implemented as computer executable code programmed by one skilled in the art to perform the method and workflow steps or functions of Figures 1 or 2. Accordingly, by executing the computer program instructions, the processor 704 executes the method and workflow steps or functions of Figures 1 or 2. Computer 702 may also include one or more network interfaces 706 for communicating with other devices via a network. Computer 702 may also include one or more input/output devices 708 that enable user interaction with computer 702 (e.g., display, keyboard, mouse, speakers, buttons, etc.).

Processor 704 may include both general and special purpose microprocessors, and may be the sole processor or one of multiple processors of computer 702. Processor 704 may include one or more central processing units (CPUs), for example. Processor 704, data storage device 712, and/or memory 710 may include, be supplemented by, or incorporated in, one or more application-specific integrated circuits (ASICs) and/or one or more field programmable gate arrays (FPGAs).

Data storage device 712 and memory 710 each include a tangible non-transitory computer readable storage medium. Data storage device 712, and memory 710, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 708 may include peripherals, such as a printer, scanner, display screen, etc. For example, input/output devices 708 may include a display device such as a cathode ray tube (CRT) or liquid crystal display (LCD) monitor for displaying information to the user, a keyboard, and a pointing device such as a mouse or a trackball by which the user can provide input to computer 702.

An image acquisition device 714 can be connected to the computer 702 to input image data (e.g., medical images) to the computer 702. It is possible to implement the image acquisition device 714 and the computer 702 as one device. It is also possible that the image acquisition device 714 and the computer 702 communicate wirelessly through a network. In a possible embodiment, the computer 702 can be located remotely with respect to the image acquisition device 714.

Any or all of the systems, apparatuses, and methods discussed herein may be implemented using one or more computers such as computer 702.

One skilled in the art will recognize that an implementation of an actual computer or computer system may have other structures and may contain other components as well, and that Figure 7 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention as defined by the claims. Those skilled in the art could implement various other feature combinations without departing from the scope of the invention as defined by the claims.

The following is a list of non-limiting illustrative embodiments disclosed herein:

Illustrative embodiment 1. A computer-implemented method comprising: receiving 1) one or more medical images of a patient and 2) a fluence map for each of a plurality of fields for radiation therapy of the patient; determining fluence-related information for each of the plurality of fields based on the fluence maps; aggregating the fluence-related information for the plurality of fields; determining a multi-field dose for the patient based on the one or more medical images and the aggregated fluence-related information using a machine learning based dose prediction network; and outputting the multi-field dose.

Illustrative embodiment 2. The computer-implemented method of illustrative embodiment 1, further comprising: determining an approximated dose for the patient based on the fluence-related information for the plurality of fields, wherein determining a multi-field dose for the patient based on the one or more medical images and the aggregated fluence-related information using a machine learning based dose prediction network comprises determining the multi-field dose for the patient further based on the approximated dose.

Illustrative embodiment 3. The computer-implemented method of illustrative embodiment 2, wherein determining a multi-field dose for the patient based on the one or more medical images and the aggregated fluence-related information using a machine learning based dose prediction network comprises: determining an initial multi-field dose based on the one or more medical images and the aggregated fluence-related information using the machine learning based dose prediction network; and combining the initial multi-field dose and the approximated dose to determine the multi-field dose for the patient.

Illustrative embodiment 4. The computer-implemented method of any one of illustrative embodiments 1-3, wherein determining a multi-field dose for the patient based on the one or more medical images and the aggregated fluence-related information using a machine learning based dose prediction network comprises: determining electron angular fluences using the machine learning based dose prediction network; and determining the multi-field dose for the patient based on the electron angular fluences.

Illustrative embodiment 5. The computer-implemented method of any one of illustrative embodiments 1-4, wherein aggregating the fluence-related information for the plurality of fields comprises: transforming a direction of the fluence-related information to coefficient vectors; scaling the coefficient vectors based on the fluence-related information; and combining the scaled coefficient vectors to provide for the aggregated fluence-related information.

Illustrative embodiment 6. The computer-implemented method of illustrative embodiment 5, wherein transforming a direction of the fluence-related information to coefficient vectors comprises: transforming the direction of the fluence-related information to coefficient vectors for each voxel of the fluence maps.

Illustrative embodiment 7. The computer-implemented method of any one of illustrative embodiments 5-6, wherein combining the scaled coefficient vectors to provide for the aggregated fluence-related information comprises: combining the scaled coefficient vectors into accumulated coefficients for each of the plurality of fields; and combining the accumulated coefficients for each of the plurality of fields into combined coefficients.

Illustrative embodiment 8. The computer-implemented method of any one of illustrative embodiments 1-7, wherein determining a multi-field dose for the patient based on the one or more medical images and the aggregated fluence-related information using a machine learning based dose prediction network comprises: determining the multi-field dose for the patient further based on a material volume of tissue to be irradiated.

Illustrative embodiment 9. The computer-implemented method of any one of illustrative embodiments 1-8, wherein the fluence-related information comprises uncollided fluence.

Illustrative embodiment 10. An apparatus comprising: means for receiving 1) one or more medical images of a patient and 2) a fluence map for each of a plurality of fields for radiation therapy of the patient; means for determining fluence-related information for each of the plurality of fields based on the fluence maps; means for aggregating the fluence-related information for the plurality of fields; means for determining a multi-field dose for the patient based on the one or more medical images and the aggregated fluence-related information using a machine learning based dose prediction network; and means for outputting the multi-field dose.

Illustrative embodiment 11. The apparatus of illustrative embodiment 10, further comprising: means for determining an approximated dose for the patient based on the fluence-related information for the plurality of fields, wherein the means for determining a multi-field dose for the patient based on the one or more medical images and the aggregated fluence-related information using a machine learning based dose prediction network comprises means for determining the multi-field dose for the patient further based on the approximated dose.

Illustrative embodiment 12. The apparatus of illustrative embodiment 11, wherein the means for determining a multi-field dose for the patient based on the one or more medical images and the aggregated fluence-related information using a machine learning based dose prediction network comprises: means for determining an initial multi-field dose based on the one or more medical images and the aggregated fluence-related information using the machine learning based dose prediction network; and means for combining the initial multi-field dose and the approximated dose to determine the multi-field dose for the patient.

Illustrative embodiment 13. The apparatus of any one of illustrative embodiments 10-12, wherein the means for determining a multi-field dose for the patient based on the one or more medical images and the aggregated fluence-related information using a machine learning based dose prediction network comprises: means for determining electron angular fluences using the machine learning based dose prediction network; and means for determining the multi-field dose for the patient based on the electron angular fluences.

Illustrative embodiment 14. The apparatus of any one of illustrative embodiments 10-13, wherein the means for determining a multi-field dose for the patient based on the one or more medical images and the aggregated fluence-related information using a machine learning based dose prediction network comprises: means for determining the multi-field dose for the patient further based on a material volume of tissue to be irradiated.

Illustrative embodiment 15. A non-transitory computer-readable storage medium comprising instructions which, when executed by a computer, cause the computer to carry out operations comprising: receiving 1) one or more medical images of a patient and 2) a fluence map for each of a plurality of fields for radiation therapy of the patient; determining fluence-related information for each of the plurality of fields based on the fluence maps; aggregating the fluence-related information for the plurality of fields; determining a multi-field dose for the patient based on the one or more medical images and the aggregated fluence-related information using a machine learning based dose prediction network; and outputting the multi-field dose.

Illustrative embodiment 16. The non-transitory computer-readable storage medium of illustrative embodiment 15, wherein aggregating the fluence-related information for the plurality of fields comprises: transforming a direction of the fluence-related information to coefficient vectors; scaling the coefficient vectors based on the fluence-related information; and combining the scaled coefficient vectors to provide for the aggregated fluence-related information.

Illustrative embodiment 17. The non-transitory computer-readable storage medium of illustrative embodiment 16, wherein transforming a direction of the fluence-related information to coefficient vectors comprises: transforming the direction of the fluence-related information to coefficient vectors for each voxel of the fluence maps.

Illustrative embodiment 18. The non-transitory computer-readable storage medium of any one of illustrative embodiments 16-17, wherein combining the scaled coefficient vectors to provide for the aggregated fluence-related information comprises: combining the scaled coefficient vectors into accumulated coefficients for each of the plurality of fields; and combining the accumulated coefficients for each of the plurality of fields into combined coefficients.

Illustrative embodiment 19. The non-transitory computer-readable storage medium of any one of illustrative embodiments 15-18, wherein determining a multi-field dose for the patient based on the one or more medical images and the aggregated fluence-related information using a machine learning based dose prediction network comprises: determining the multi-field dose for the patient further based on a material volume of tissue to be irradiated.

Illustrative embodiment 20. The non-transitory computer-readable storage medium of any one of illustrative embodiments 15-19, wherein the fluence-related information comprises uncollided fluence.

**Claims**

1. A computer-implemented method comprising:

   receiving (102) 1) one or more medical images (202) of a patient and 2) a fluence map (204) for each of a plurality of fields for radiation therapy of the patient;
   determining (104) fluence-related information (206) for each of the plurality of fields based on the fluence maps;
   aggregating (108) the fluence-related information for the plurality of fields; determining (110) a multi-field dose (216) for the patient based on the one or more medical images and the aggregated fluence-related information (212) using a machine learning based dose prediction network (214); and
   outputting (112) the multi-field dose.

2. The computer-implemented method of claim 1, further comprising:

   determining an approximated dose (106) for the patient based on the fluence-related information for the plurality of fields,
   wherein determining a multi-field dose for the patient based on the one or more medical images and the aggregated fluence-related information using a machine learning based dose prediction network comprises determining the multi-field dose for the patient further based on the approximated dose.

3. The computer-implemented method of claim 2, wherein determining a multi-field dose for the patient based on the one or more medical images and the aggregated fluence-related information using a machine learning based dose prediction network comprises:

determining an initial multi-field dose based on the one or more medical images and the aggregated fluence-related information using the machine learning based dose prediction network; and
combining the initial multi-field dose and the approximated dose to determine the multi-field dose for the patient.

4. The computer-implemented method of any one of claims 1 - 3, wherein determining a multi-field dose for the patient based on the one or more medical images and the aggregated fluence-related information using a machine learning based dose prediction network comprises:

determining electron angular fluences using the machine learning based dose prediction network; and
determining the multi-field dose for the patient based on the electron angular fluences.

5. The computer-implemented method of any one of claims 1 - 4, wherein aggregating the fluence-related information for the plurality of fields comprises:

transforming a direction of the fluence-related information to coefficient vectors;
scaling the coefficient vectors based on the fluence-related information; and
combining the scaled coefficient vectors to provide for the aggregated fluence-related information.

6. The computer-implemented method of claim 5, wherein transforming a direction of the fluence-related information to coefficient vectors comprises:
transforming the direction of the fluence-related information to coefficient vectors for each voxel of the fluence maps.

7. The computer-implemented method of claim 5 or 6, wherein combining the scaled coefficient vectors to provide for the aggregated fluence-related information comprises:

combining the scaled coefficient vectors into accumulated coefficients (210) for each of the plurality of fields; and
combining the accumulated coefficients for each of the plurality of fields into combined coefficients.

8. The computer-implemented method of any one of claims 1 - 7, wherein determining a multi-field dose for the patient based on the one or more medical images and the aggregated fluence-related information using a machine learning based dose prediction network comprises:
determining the multi-field dose for the patient further based on a material volume of tissue to be irradiated.

9. The computer-implemented method of any one of claims 1 - 8, wherein the fluence-related information comprises uncollided fluence (206).

10. An apparatus comprising:

means for receiving (102) 1) one or more medical images (202) of a patient and 2) a fluence map (204) for each of a plurality of fields for radiation therapy of the patient;
means for determining (104) fluence-related information (206) for each of the plurality of fields based on the fluence maps;
means for aggregating (108) the fluence-related information for the plurality of fields;
means for determining (110) a multi-field dose (216) for the patient based on the one or more medical images and the aggregated fluence-related information (212) using a machine learning based dose prediction network (214); and
means for outputting (112) the multi-field dose.

11. The apparatus of claim 10, further comprising:

means for determining an approximated dose (106) for the patient based on the fluence-related information for the plurality of fields,
wherein the means for determining a multi-field dose for the patient based on the one or more medical images and the aggregated fluence-related information using a machine learning based dose prediction network comprises

means for determining the multi-field dose for the patient further based on the approximated dose.

12. The apparatus of claim 11, wherein the means for determining a multi-field dose for the patient based on the one or more medical images and the aggregated fluence-related information using a machine learning based dose prediction network comprises:

means for determining an initial multi-field dose based on the one or more medical images and the aggregated fluence-related information using the machine learning based dose prediction network; and
means for combining the initial multi-field dose and the approximated dose to determine the multi-field dose for the patient.

13. The apparatus of any one of claims 10 - 12, wherein the means for determining a multi-field dose for the patient based on the one or more medical images and the aggregated fluence-related information using a machine learning based dose prediction network comprises:

means for determining electron angular fluences using the machine learning based dose prediction network; and
means for determining the multi-field dose for the patient based on the electron angular fluences.

14. The apparatus of any one of claims 10 - 13, wherein the means for determining a multi-field dose for the patient based on the one or more medical images and the aggregated fluence-related information using a machine learning based dose prediction network comprises:
means for determining the multi-field dose for the patient further based on a material volume of tissue to be irradiated.

15. A computer program product comprising instructions which, when executed by a computer, cause the computer to carry out the method according to any one of claims 1 - 9.

# FIG. 1

<u>100</u>

Receive 1) one or more medical images of a patient and 2) a fluence map
for each of a plurality of fields for radiation therapy of the patient
<u>102</u>

Determine fluence-related information for each of the plurality of fields
based on the fluence maps
<u>104</u>

Determine an approximated dose for the patient based on the fluence-
related information for the plurality of fields
<u>106</u>

Aggregate the fluence-related information for the plurality of fields
<u>108</u>

Determine a multi-field dose for the patient based on the one or more
medical images, the aggregated fluence-related information, and the
approximated dose using a machine learning based dose prediction
network
<u>110</u>

Output the multi-field dose
<u>112</u>

FIG. 2

EP 4 717 305 A1

FIG. 3

FIG. 4

FIG. 5

FIG. 6

EP 4 717 305 A1

# FIG. 7

702

Network interface 706

I/O 708

Processor 704

Storage 712

Memory 710

Image Acquisition Device 714

Europäisches
Patentamt

European
Patent Office

Office européen
des brevets

# EUROPEAN SEARCH REPORT

**Application Number**

**EP 25 20 3539**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/088410 A1 (HIBBARD LYNDON STANLEY [US]) 24 March 2022 (2022-03-24) <br> * paragraphs [0002], [0008], [0043] - [0056], [0102] - [0111], [0119] - [0124], [0137] - [0139] * <br> ----- | 1-15 | INV. <br> A61N5/10 |
| X | US 2020/398079 A1 (ADELSHEIM CHARLES [US] ET AL) 24 December 2020 (2020-12-24) <br> * paragraphs [0017] - [0026], [0034] - [0036], [0081] - [0092], [0102] - [0109] * <br> ----- | 1-15 | |

TECHNICAL FIELDS
SEARCHED (IPC)

A61N

The present search report has been drawn up for all claims

| Place of search | Date of completion of the search | Examiner |
|---|---|---|
| Munich | 14 January 2026 | Lohmann, Stefan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 .......................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 20 3539

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2026

| Patent document cited in search report | Publication date | Patent family member(s) | | Publication date |
|---|---|---|---|---|
| US 2022088410 A1 | 24-03-2022 | CN | 116391234 A | 04-07-2023 |
| | | EP | 4200872 A1 | 28-06-2023 |
| | | US | 2022088410 A1 | 24-03-2022 |
| | | WO | 2022061324 A1 | 24-03-2022 |
| US 2020398079 A1 | 24-12-2020 | CN | 113795303 A | 14-12-2021 |
| | | EP | 3986547 A1 | 27-04-2022 |
| | | US | 2020398079 A1 | 24-12-2020 |
| | | WO | 2020257216 A1 | 24-12-2020 |

EPO FORM P0459

For more details about this annex : see Official Journal of the European Patent Office, No. 12/82